(19) Europäisches Patentamt
European Patent Office
Office européen des brevets

(11) EP 4 241 825 A1

(12) EUROPÄISCHE PATENTANMELDUNG

(43) Veröffentlichungstag:
13.09.2023 Patentblatt 2023/37

(51) Internationale Patentklassifikation (IPC):
**A61M 37/00** (2006.01)

(21) Anmeldenummer: 22160536.3

(52) Gemeinsame Patentklassifikation (CPC):
**A61M 37/0076**

(22) Anmeldetag: 07.03.2022

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **MT.DERM**
**12307 Berlin (DE)**

(72) Erfinder: **SCHERKOWSKI, Dirk**
**12489 Berlin (DE)**

(74) Vertreter: **Bittner, Thomas L.**
**Boehmert & Boehmert**
**Anwaltspartnerschaft mbB**
**Pettenkoferstrasse 22**
**80336 München (DE)**

(54) **MICRONEEDLING-HANDGERÄT ZUM LOKALEN AUFSTECHEN EINER HAUT UND HAUTSTECHEINRICHTUNG**

(57) Die Erfindung betrifft ein Microneedling-Handgerät (1) zum lokalen Aufstechen einer Haut mit einem von einem Nutzer greifbaren Handstück (7) und einer Microneedling-Hautstecheinrichtung, die einen Nadelträger (11) mit einer Anordnung von Stechnadeln (6) aufweist, bei der über einen Flächenabschnitt (12) des Nadelträgers (11) verteilt mit Abstand voneinander mehrere flexibel biegsame Stechnadeln (6) angeordnet sind, wobei ein in Bezug auf den Flächenabschnitt (12) proximales Nadelende (13) der Stechnadeln (6) an dem Nadelträger (11) fixiert ist und die Stechnadeln (6) jeweils aus einem Vollmaterial bestehen. Weiterhin ist eine Microneedling-Hautstecheinrichtung für ein Microneedling-Handgerät geschaffen.

Fig. 2

**Beschreibung**

Die Erfindung betrifft ein Microneedling-Handgerät zum lokalen Aufstechen einer Haut sowie eine Hautstecheinrichtung für ein Microneedling-Handgerät.

Hintergrund

Beim Microneedling wird die Haut mit mehreren Stechnadeln in einem flächigen Hautabschnitt gleichzeitig lokal aufgestochen. Die mehreren Stechnadeln sind an einem Microneedling-Stechwerkzeug eines Handgeräts über eine Fläche verteilt angeordnet, zum Beispiel an einer Nadelplatte.

Das Microneedling verursacht aufgrund des lokalen Aufstechens feinste Verletzungen der Haut. In der Haut werden dann verschiedene Botenstoffe und Wachstumsfaktoren ausgeschüttet, um die Wundheilung anzuregen. Dieser Vorgang regt auch die Produktion von Kollagen, Elastin und Hyaluronsäure an, die Straffung und Elastizität der Haut fördern. Microneedling kann mit und ohne Substanz- oder Wirkstoffeintrag in die Haut ausgeführt werden. Die Haut wird lokal aufgestochen, um positive Heilungsreaktionen auszulösen, was wahlweise mittels Substanzeintrag unterstützt werden kann.

Zum Anwenden des Microneedling sind beispielsweise manuelle Hautstecheinrichtungen in stempelartiger Ausführung bekannt, bei denen das Microneedling-Stechwerkzeug mit einem Stempel gebildet ist, an dem über eine Stempelfläche verteilt mehrere Stechnadeln angeordnet sind. Bei anderen manuellen Handgeräten sind die Stechnadeln über die Mantelfläche einer Walze verteilt, die zum lokalen Aufstechen der Haut über diese gerollt wird.

Weiterhin sind Microneedling-Handgeräte in Form eines sogenannten Pens bekannt, bei dem ein Microneedling-Stechwerkzeug einer Hautstecheinrichtung mit einer Nadelplatte oder einem Nadelträger gebildet ist, an der / dem die mehreren Stechnadeln angeordnet sind. Das Microneedling-Stechwerkzeug wird mit Hilfe eines elektrischen Motors einer Antriebseinrichtung automatisiert vor- und zurückbewegt. Mit Hilfe des elektrischen Motors der Antriebseinrichtung wird eine Antriebskraft bereitgestellt, die auf das Microneedling-Stechwerkzeug eingekoppelt wird, um die Nadelplatte und den hieran flächig verteilten Stechnadeln vor und zurück zu bewegen, derart, dass Nadelspitzen der Stechnadeln in Bezug auf eine vorderseitige Gehäuseöffnung eines Gehäuses des Handgeräts zwischen einer ausgefahrenen und einer eingefahrenen Stellung vor- und zurückbewegt werden. Eine Nadelplatte, an welcher die Stechnadeln angeordnet sind, ist von einem vorderen Gehäuseabschnitt umgeben. Ein vorderseitiger Rand des Gehäuseabschnitts, welcher die Gehäuseöffnung umgibt, durch welche hindurch die Nadelspitzen der Stechnadeln aus- und eingefahren werden, wird im Betrieb auf die Hautoberfläche aufgelegt, welche hierdurch in dem Anwendungsbereich fixiert wird, um dann mittels Vor- und Zurückbewegens der Nadelspitzen die (gespannte) Hautoberfläche lokal aufzustechen. Der die vorderseitige Gehäuseöffnung umgebende Rand dient hier dem Fixieren und Festhalten des Hautabschnitts, in welchen dann lokal mittels der Stechnadeln an der Nadelplatte eingestochen wird.

Zusammenfassung

Aufgabe der Erfindung ist es, ein Microneedling-Handgerät zum lokalen Aufstechen einer Haut sowie eine Hautstecheinrichtung für ein Microneedling-Handgerät anzugeben, welche ein verbessertes Anwenden des Microneedling ermöglichen.

Gelöst wird die Aufgabe durch ein Microneedling-Handgerät zum lokalen Aufstechen einer Haut nach Anspruch 1. Weiterhin ist eine Hautstecheinrichtung für ein Microneedling-Handgerät nach dem nebengeordneten Anspruch 15 geschaffen. Ausgestaltungen sind Gegenstand von abhängigen Unteransprüchen.

Nach einem Aspekt ist ein Microneedling-Handgerät zum lokalen Aufstechen einer Haut geschaffen, welches Folgendes aufweist: ein von einem Nutzer greifbares Handstück und eine Microneedling-Hautstecheinrichtung, die einen Nadelträger mit einer Anordnung von Stechnadeln aufweist, bei der über einen Flächenabschnitt des Nadelträgers verteilt mit Abstand voneinander mehrere flexibel biegsame Stechnadeln angeordnet sind, wobei ein in Bezug auf den Flächenabschnitt proximales Nadelende der Stechnadeln am Nadelträger fixiert ist und die Stechnadeln jeweils aus einem Vollmaterial bestehen.

Nach einem weiteren Aspekt ist eine Microneedling-Hautstecheinrichtung für ein Microneedling-Handgerät geschaffen, welche als Wechselmodul ausgeführt ist und die mit einem Nadelträger mit einer Anordnung von Stechnadeln gebildet ist, bei der über einen Flächenabschnitt des Nadelträgers verteilt mit Abstand voneinander mehrere flexibel biegsame Stechnadeln angeordnet sind, wobei ein in Bezug auf den Flächenabschnitt proximales Nadelende der Stechnadeln an dem Nadelträger fixiert ist und die Stechnadeln jeweils aus einem Vollmaterial bestehen.

Die Anordnung und die Ausbildung der Stechnadeln an der Microneedling-Hautstecheinrichtung ermöglichen ein elastisches Biegen der Stechnadeln beim Aufsetzen einer jeweiligen Nadelstechspitze an einem distalen Nadelende der Stechnadeln in einem zu behandelnden Hautbereich. Wie die proximalen Nadelenden sind auch die distalen Nadelenden der Stechnadeln zumindest im nicht belasteten oder nicht gebogenen Zustand voneinander beabstandet. Im Anwendungsfall biegen sich die Stechnadeln beim Aufsetzen auf einer zu behandelnden Hautoberfläche, so dass die Stechnadeln jeweils einen gekrümmten oder gebogenen Nadelabschnitt aufweisen, welcher die Stechnadeln wahlweise im Wesentlichen über ihre gesamte Nadellänge erfassen kann.

Die Stechnadeln können an dem Nadelträger fest oder lösbar angeordnet sein. In einem Ausführungsbeispiel kann vorgesehen sein, dass die Stechnadeln mit ihrem proximalen Ende in einem Nadeltragelement fest angeordnet sind, welches seinerseits in einer zugeordneten Aufnahme an dem Nadelträger lösbar angeordnet ist, zum Beispiel mittels Einsteckens. Alternativ können die Stechnadeln frei von einem solchen Nadeltragelement in eine zugeordnete Aufnahme an dem Nadelträger lösbar eingesteckt sein.

Die Stechnadeln können zum Beispiel aus Metall bestehen.

Der Nadelträger kann in Bezug auf eine Längsrichtung des Handgeräts und / oder eine Längsrichtung der Stechnadeln im nicht gebogenen Zustand geneigt angeordnet sein. Beispielsweise kann eine Oberfläche des Flächenabschnitts des Nadelträgers, in welchem die Stechnadeln am Nadelträger angeordnet sind, zu der einen und / oder der anderen Längsrichtung geneigt ausgebildet sein.

Die Stechnadeln können einen Durchmesser von höchstens etwa 0,20 mm aufweisen. In einem Ausführungsbeispiel weisen die Stechnadeln einen Durchmesser von höchstens etwa 0,15mm auf. Diese Durchmesser können insbesondere bei Stechnadeln aus Metall vorgesehen sein, zum Beispiel Stechnadeln aus Edelstahl.

Die Stechnadeln können wenigstens so biegsam ausgeführt sein, dass sie sich beim Aufsetzen auf die Haut bereits biegen und in der Epidermis der Haut stecken bleiben. Die Stechnadeln können über ihre gesamte Nadellänge so torsionssteif sein, dass ein im Betrieb aufgrund einer Stechnadel-Haut-Interaktion via Reibung eingebrachtes Torsionsmoment trotz ausgelenkter oder gebogener Stechnadel überwunden wird.

Die Stechnadeln können einen im Wesentlichen kreisrunden Querschnitt aufweisen.

Die Stechnadeln können voneinander einen Abstand von wenigstens etwa 1 mm aufweisen, insbesondere im Bereich des Flächenabschnitts am Nadelträger. Ein maximaler Abstand der Stechnadeln voneinander kann etwa 5 mm betragen.

Die Stechnadeln können eine Nadellänge zwischen etwa 1 mm und etwa 100 mm aufweisen. In einer Ausgestaltung können die Stechnadeln eine Nadellänge zwischen etwa 5 mm und etwa 60 mm aufweisen, alternativ eine Nadellänge zwischen etwa 10 mm und etwa 40 mm.

Die Anordnung von Stechnadeln kann wenigstens fünf Stechnadeln im Bereich der Anwendungsfläche aufweisen.

Die jeweiligen Längsachsen der Stechnadeln können zumindest in einer nichtgebogenen Stellung der Stechnadeln im Wesentlich parallel zueinander verlaufen.

Das Verhalten der Stechnadeln im Betrieb, wenn das Microneedling-Handgerät auf einen zu bearbeitenden Hautabschnitt aufgesetzt ist, ist jeweils gleich, derart, dass ein Muster der Anordnung der Stechnadeln im Bereich der proximalen Einspannung auch in Haut-parallelen Querschnittsebenen im Wesentlichen erhalten bleibt.

Die Stechnadeln können in der nichtgebogenen Stellung einer Bauform aus der folgenden Gruppe entsprechend gebildet sind: gerade Nadel, schlangenlinienförmige Nadel und gekrümmte Nadel.

Die Stechnadeln können ausgebildet sein

i) mit einer kritischen Knicklast $F_{kritisch}$

$$F_{kritisch} = \frac{2\pi^2}{L^2} \cdot I \cdot E$$

und

ii) mit einer Knicklänge s

$$s = \frac{1}{2}\sqrt{2} \cdot L \,,$$

wobei L die Nadellänge der Stechnadel, E das Elastizitätsmodul für ein Material der Stechnadel und $I$ das axiale Flächenträgheitsmoment des Querschnitts der Stechnadel angeben. Eine solche Ausbildung der Stechnadeln entspricht einem der an sich bekannten sogenannten EULER'schen Knickfälle, insbesondere Fall 3. Die kritische Knicklast kann mit der tatsächlich im Betrieb vorhandenen Last ($F_{real}$) ein Verhältnis von kleinergleich 1 ausbilden (($F_{real}$ / $F_{kritisch}$) ≤ 1), vorzugsweise deutlich darunter.

Um für eine beispielhaft vorhandene oder gegebene Stechnadel ($L_0$, $d_0$) mit kreisrunder Querschnittsform abzuschätzen, wie für diese, unter Beibehaltung des Knickfalls (insbesondere Fall 3) und des Nadelmaterials, zum Beispiel Edelstahl, konstruktive Eigenschaften der Stechnadel, wie Länge (L) und Durchmesser (d), in Bereichen abgewandelt werden können, um immer noch eine geeignete, aber abgewandelte Stechnadel 1 ($L_1$, $d_1$) bei gleicher kritischer Knicklast für das Microneedling-Handgerät oder die Microneedling-Hautstecheinrichtung bereitzustellen, vereinfacht sich die Formel mittels Gleichsetzen:

$$\frac{d_1^4}{d_0^4} = \frac{L_1^2}{L_0^2}$$

Diese kann leicht nach einem freien Parameter umgestellt werden, um Abschätzungen für die geeignete konstruktive Ausgestaltung der Stechnadeln zu bestimmen. Im Beispielfall des EULER'schen Knickfalls 3 und einer kreisrunden Querschnittsform der Stechnadel ergibt sich, ausgehend von einer ersten gegebenen (biegsamen) Stechnadel (Musternadel 1) mit $L_1$ = 15mm und $d_1$= 0,1mm, für weitere abgewandelte Stechnadeln 4 und 5 ($L_4$, $d_4$; $L_5$, $d_5$) mit $L_4$ = 30mm bzw. $d_5$= 0,12mm Folgendes: (i) $d_4$= 0,141mm und (ii) $L_5$ = 21,6mm.

Im Fall einer beispielhaften zweiten gegebenen (biegsamen) Stechnadel (Musternadel 2) mit $L_2$ = 30mm und $d_2$ = 0,12mm ergibt sich, hiervon ausgehend, für die zwei weiteren abgewandelten Stechnadeln 6 und 7 ($L_6$, $d_6$; $L_7$, $d_7$) mit $L_6$ = 15mm und $d_7$= 0,1mm Folgendes: (iii) $d_6$= 0,085mm und (iv) $L_7$ = 20,8mm.

Zunächst wurde für einen Musternadel-Vergleich eine Vergleichsnadel mit den Parametern der jeweils anderen Musternadel gesucht und jeweils einmal die Länge und einmal der Durchmesser vorgegeben. Anhand der Ergebnisse lässt sich erkennen, dass die Musternadel 2 die höhere Biegsamkeit der beiden Musternadeln 1 und 2 aufweist.

Hilfreich können auch vergleichende Abschätzungen zu Stechnadeln sein, die bei Handgeräten im Permanent-Make-Up-Bereich oder Tattoo-Anwendungsbereich üblicherweise eingesetzt werden und die sich dort beim lokalen Aufstechen der Haut im Betrieb gerade nicht biegen sollen. Eine im Permanent-Make-Up-Bereich oder Tattoo-Anwendungsbereich eingesetzte erste Stechnadel (für einen nicht-biegenden oder biegefesten Einsatz) desselben Materials (zum Beispiel medizinischer Edelstahl), die sich bei der Anwendung auf die (aufzustechende) Haut nicht oder unmerklich biegt und zum direkten Stechen eingerichtet ist, besitzt beispielsweise einen Durchmesser von 0,3mm und eine Länge von etwa 35mm, wobei dort ein anderer Knickfall (EULER'scher Knickfall 4) mit deutlich steiferer Führung vorliegen kann, weil die Einzelstechnadel an der Stechspitze in einem engen Röhrchen geführt ist (insbesondere bei einer Modulspitze mit vorderer Modulöffnung). Um ausgehend von den Parametern für diese bekannte Stechnadel, welche nicht für das hier beschriebene Microneedling-Handgerät eingerichtet ist, eine geeignete Stechnadel für das Microneedling-Handgerät zu schaffen, ergibt sich unter Verwendung der obigen vereinfachten Formel eine deutliche Abweichung der Ergebnisse: Um gleich biegsam zu sein wie Musternadel 2 müsste die Länge 187,5mm (bei d = 0,3mm) betragen, im Vergleich zu Musternadel 1 immerhin noch 135mm (bei $d$ = 0,3mm).

Eine im Permanent-Make-Up-Bereich oder Tattoo-Anwendungsbereich eingesetzte zweite Stechnadel (für einen nicht-biegenden oder biegefesten Einsatz), die beim direkten Stechen ohne nennenswerte Biegung reagiert, weist einen Durchmesser von 0,18mm und eine Länge von 35mm auf. Hiervon ausgehend ergibt sich unter Verwendung der obigen vereinfachten Formel: eine Länge von 67,5mm für eine biegsame Vergleichsnadel hinsichtlich Musternadel 2 und eine Länge von 48,6mm hinsichtlich Musternadel 1.

Diese Längen-Abschätzungen für die Beispielstechnadeln ($\varnothing$0,3×35 und $\varnothing$0,18×35) zeigen, dass die im Permanent-Make-Up-Bereich oder Tattoo-Anwendungsbereich üblicherweise eingesetzten Stechnadeln sich von den biegsamen Nadeln deutlich unterscheiden und abgrenzen lassen. Selbige Abschätzungen ließen sich für gegebene Längen und gesuchte Durchmesser wiederholen. Im Umkehrschluss kann die Biegsamkeit einer neuen Nadel anhand der bekannten Werte bestimmt werden.

So kann zum Beispiel die bekannte, gerade noch biegesteife Nadel vom Durchmesser 0,18mm und der Länge 35mm als weitere Musternadel 3 (Grenznadel A) herangezogen werden, um hiervon ausgehend iterativ eine Stechnadel für das hier offenbarte Microneedling-Handgerät oder die Microneedling-Hautstecheinrichtung zu bestimmen. Beim Durchmesser wurden die Werte der bekannten geeigneten Biegenadeln (= Musternadeln 1 und 2) vorgegeben. Damit wurde ermittelt, dass eine biegeweiche Stechnadel zweckmäßig vom Durchmesser 0,1mm mindestens länger als 10,8mm und vom Durchmesser 0,12mm mindestens länger als 15,5mm ist. Das erste Ergebnis liegt in der Nähe der Musternadel 1 ($\varnothing$0,1 × 12mm), die als die steifere Biegenadel (Grenznadel B) identifiziert wurde. Demnach kann in dem betrachteten Beispiel eine "Master-Stechnadel" dazwischen, also mit den Abmessungen $\varnothing$0,1 × 11mm als obere Grenze angenommen werden: alle Stechnadeln sollen biegsamer sein als diese.

Die Microneedling-Hautstecheinrichtung kann mit dem greifbaren Handstück lösbar verbunden sein. Bei dieser oder anderen Ausführungsformen kann die Microneedling-Hautstecheinrichtung als ein Einwegmodul ausgeführt sein. Alternativ kann vorgesehen sein, dass die Microneedling-Hautstecheinrichtung sterilisierbar ist, so dass nach einer Verwendung des Microneedling-Handgeräts für eine Hautbehandlung eine Sterilisation der Stechnadeln vorgenommen werden kann, um das Handgerät für eine weitere Anwendung vorzubereiten.

Der Nadelträger kann lösbar an der Microneedling-Hautstecheinrichtung angeordnet sein.

Zumindest distale Stechenden der Stechnadeln können in einer Schutzkappe angeordnet sein, welche lösbar ist. Mit Hilfe der Schutzkappe sind unbeabsichtigte Verletzungen durch die Stechnadeln verhindert. Wenn das Microneedling-Handgerät zur Behandlung verwendet werden soll, wird die Schutzkappe abgenommen, so dass zumindest die distalen Stechenden der Stechnadeln frei liegen.

Die Schutzkappe kann nach dem Lösen und dem Freigeben der distalen Stechenden rückseitig an der Microneedling-Hautstecheinrichtung anordenbar und so das greifbare Handstück zumindest teilweise ausbildend ausgeführt sein. Die Schutzkappe ist hierbei multifunktionell ausgebildet, derart, dass sie ergänzend dazu nutzbar ist, das greifbare Handstück auszubilden oder zu erweitern. Hierzu ist die Schutzkappe zum Beispiel eingerichtet, rückseitig auf die Microneedling-Handstecheinrichtung aufgesteckt oder aufgeschraubt zu werden. Hierdurch kann beispielsweise eine Verlängerung ausbildet werden, die der Nutzer dann mit den Fingern greifen kann.

Die Schutzkappe kann ein oder mehrere Durchbrüche aufweisen, welche für eine Sterilisation der Stechnadeln durch den einen oder die mehrere Durchbrüche hindurch nutzbar sind.

Das Microneedling-Handgerät kann eine Antriebseinrichtung aufweisen, die in einem Gehäuse angeordnet und eingerichtet ist, eine Antriebskraft bereitzustellen, und die mit der Microneedling-Hautstecheinrichtung verbunden ist, derart, dass der Nadelträger mit der Anordnung von Stechnadeln mittels der auf den Nadelträger eingeleiteten Antriebskraft eine ellipsen- oder kreisförmige Bewegung in einer Bewegungsebene quer zur jeweiligen Längsachse der Stechnadel ausführt. Mit Hilfe der Antriebskraft ist der Nadelträger mit der hieran angeordneten Anordnung von Stechnadeln im Betrieb um eine Drehachse drehbar. Nachdem die Nadeln auf eine zu behandelnde Hautfläche aufgesetzt und durch den Anpressdruck gebogen sind, werden die gebogenen Nadeln dann aufgrund der Drehung des Nadelträgers umlaufend bewegt, insbesondere entlang einer Kegel- oder Kreiseloberfläche.

An der Schutzkappe und / oder dem Handstück können ein oder mehrere funktionelle Elemente vorgesehen sein, zum Beispiel einstückig angeformt. Beispielsweise kann eine optische Lupe vorgesehen sein. Alternativ oder ergänzend kann an der Schutzkappe und / oder dem Handstück ein Spatel, wenigstens mindestens eine Spitze, ein Hautstimulator und / oder ein anderes Werkzeug vorgesehen sein, insbesondere zur Hautbehandlung und / oder - manipulation. Auch eine oder mehrere Aufnahmen für eine Flüssigkeit, die vor, während und / oder nach einer Microneedling-Behandlung zu applizieren ist, sind denkbar, ebenso alternativ oder ergänzend ein Werkzeug oder steriles Zubehör, beispielsweise Zahnstocher, feines Garn oder eine Schlaufe für Haarentfernung oder Hautpflege oder dergleichen.

Bei dem Microneedling-Handgerät kann in dem das Handstück bereitstellenden Gehäuse die Antriebseinrichtung in Form eines elektrischen Motors angeordnet sein, welcher zum Beispiel Energie von Batterien empfängt. Die Antriebseinrichtung kann vorderseitig ein Exzenterbauteil aufweisen, welches an den Nadelträger mit der Anordnung von Stechnadeln koppelt, derart, dass der Nadelträger aufgrund der eingekoppelten Antriebskraft und einer Zwangs-Gleitführung in xy-Richtung eine ellipsen- oder kreisförmige Bewegung frei von einer Rotation in einer Bewegungsebene quer zur Längsrichtung des Gehäuses (z-Richtung) ausführt.

Das Microneedling-Handgerät kann eine Nadelführungskappe mit einer vorderen Öffnung aufweisen, die im Betrieb auf eine zu behandelnde Hautfläche aufgesetzt wird. Hierbei biegen sich dann die in der Nadelführungskappe aufgenommenen Stechnadeln elastisch und flexibel innerhalb der Nadelführungskappe. Zur Führung können die gebogenen Nadeln zumindest abschnittsweise innen an der Nadelführungskappe anliegen. Wird sodann der Nadelträger mit den hieran fixierten proximalen Nadelenden der Stechnadeln aufgrund der eingeleiteten Antriebskraft in der Bewegungsebene quer zur Längsrichtung bewegt, sind die Stechnadeln eingerichtet, dass die proximalen Nadelenden der Stechnadeln in Nadellängsrichtung quasi auf einer Kreisbahn um den jeweiligen Einstechpunkt umlaufen.

Im nicht auf die Hautoberfläche aufgesetzten Zustand können die Stechnadeln zumindest innerhalb der Nadelführungskappe nicht gebogen (gerade) sein. Stechnadelspitzen sind auch in dieser geraden Stellung der Stechnadeln außerhalb vor der Öffnung angeordnet.

Mittels einer wahlweise vorgesehen Einrichtung, die innerhalb der Hautauflagefläche / Nadelaustrittsöffnung angeordnet ist, können die Stechnadeln einem Muster entsprechend so geordnet werden, dass die Nadelspitzen beim Aufsetzen auf die zu behandelnde Hautfläche einerseits das Muster der Stechnadelanordnung wiederholen und andererseits zum Ende des Aufsetzvorgangs annähernd in einer gedachten Mitte der umlaufenden Kreisbewegung zu liegen kommen. Bei dieser Ausführungsform können die Stechnadeln auch in der nicht auf die behandelnde Hautfläche aufgesetzten Stellung leicht gebogen sein.

Die Nadelführungskappe kann umlaufend mit einer gekrümmten Wandung ausgebildet sein, zum Beispiel mit einer Ball- oder Kugelform.

Die vorangehend erläuterten Ausgestaltungen können im Zusammenhang mit der Microneedling-Hautstecheinrichtung entsprechend vorgesehen sein.

Beschreibung von Ausführungsbeispielen

Nachfolgend werden weitere Ausführungsbeispiele unter Bezugnahme auf Figuren einer Zeichnung erläutert. Hierbei zeigen:

Fig. 1    eine schematische perspektivische Darstellung einer manuellen Ausführung eines Microneedling-Handgeräts zum lokalen Aufstechen einer Haut;

Fig. 2    eine schematische perspektivische Darstellung des Microneedling-Handgeräts aus Fig. 1 mit teilweise abgenommener Schutzkappe;

Fig. 3.    eine schematische perspektivische Darstellung des Microneedling-Handgeräts aus Fig. 1 mit vollständig abgenommener Schutzkappe;

Fig. 4    eine schematische perspektivische Darstellung des Microneedling-Handgeräts aus Fig. 1, wobei die Schutzkappe getrennt gezeigt ist;

Fig. 5    eine schematische perspektivische Darstellung des Microneedling-Handgeräts aus Fig. 1 vor dem rückseitigen Aufsetzen der Schutzkappe;

Fig. 6    eine schematische perspektivische Darstellung des Microneedling-Handgeräts aus Fig. 1 mit rückseitig aufgesetzter Schutzkappe;

Fig. 7     eine schematische perspektivische Darstellung des Microneedling-Handgeräts aus Fig. 1 für einen Betrieb ohne rückseitig aufgesetzte Schutzkappe;

Fig. 8     eine schematische perspektivische Darstellung eines anderen Microneedling-Handgeräts in maschinell angetriebener Ausführung;

Fig. 9     eine schematische perspektivische Schnittdarstellung des anderen Microneedling-Handgeräts aus Fig. 8 im aufgesetzten Zustand;

Fig. 10     eine schematische perspektivische Schnittdarstellung eines vorderen Teilabschnitts des weiteren Microneedling-Handgeräts aus Fig. 8 im nicht aufgesetzten Zustand;

Fig. 11     eine schematische Darstellung einer weiteren manuellen Ausführungsform für ein Microneedling-Handgerät zum lokalen Aufstechen einer Haut;

Fig. 12     eine schematische Darstellung einer anderen manuellen Ausführungsform für ein Microneedling-Handgerät zum lokalen Aufstechen einer Haut und

Fig. 13     eine schematische Darstellung einer zusätzlichen manuellen Ausführungsform für ein Microneedling-Handgerät zum lokalen Aufstechen einer Haut.

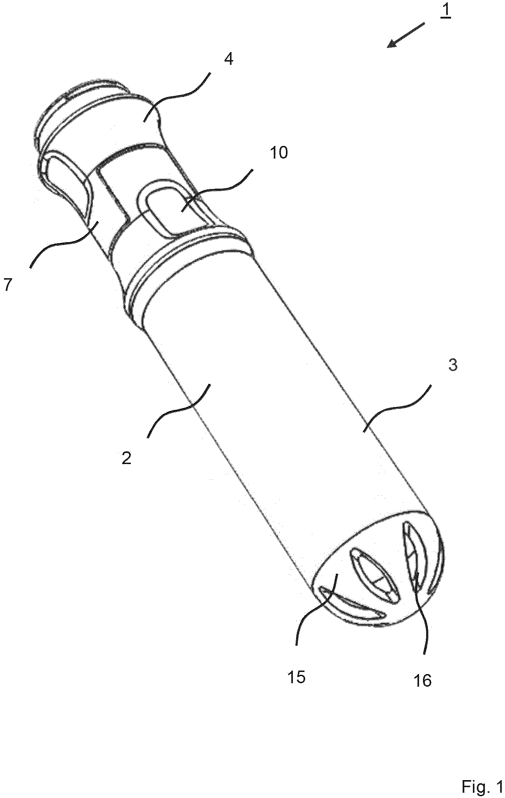

In den Fig. 1 bis 7 sind perspektivische Darstellungen eines Microneedling-Handgeräts 1 in manueller Ausführung gezeigt. Das Microneedling-Handgerät 1 weist ein Gehäuse 2 mit einer Schutzkappe 3 und einem Basisbauteil 4 auf. Die Schutzkappe 3 ist lösbar an einer zugeordneten Schutzkappenaufnahme 5a an dem Basisbauteil 4 angeordnet.

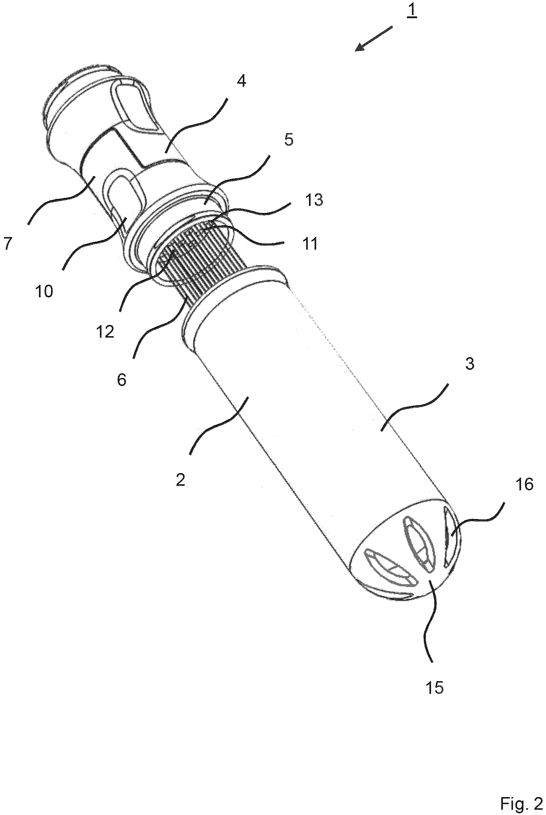

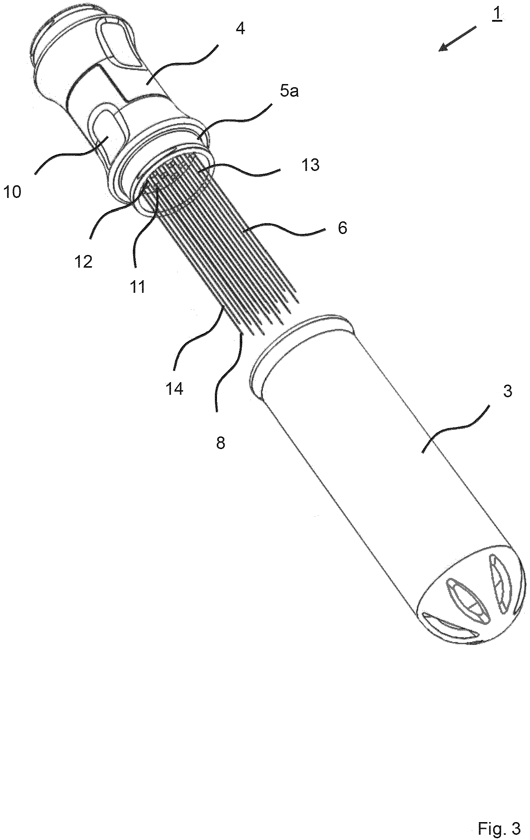

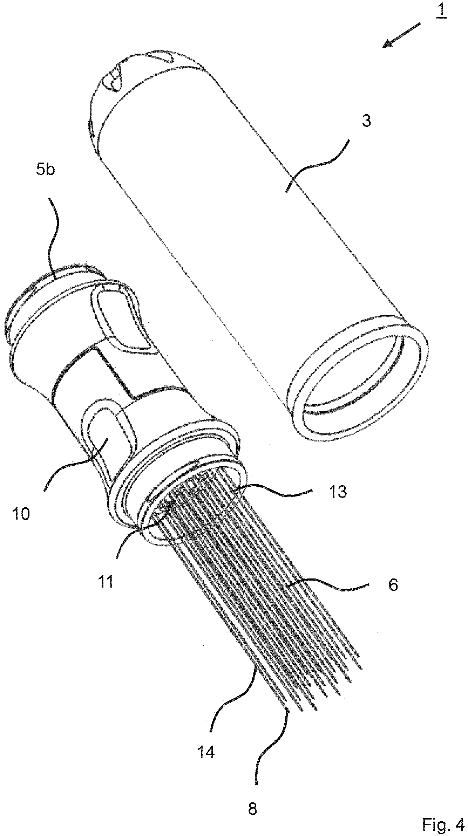

Gemäß den Fig. 2 bis 4 kann die Schutzkappe 3 abgenommen werden, indem zum Beispiel eine Steck- oder eine Schraubverbindung zum Basisbauteil 4 gelöst wird. Nach dem Abnehmen der Schutzkappe 3 liegt eine Anordnung von Stechnadeln 6 frei (vgl. insbesondere Fig. 3 und 4).

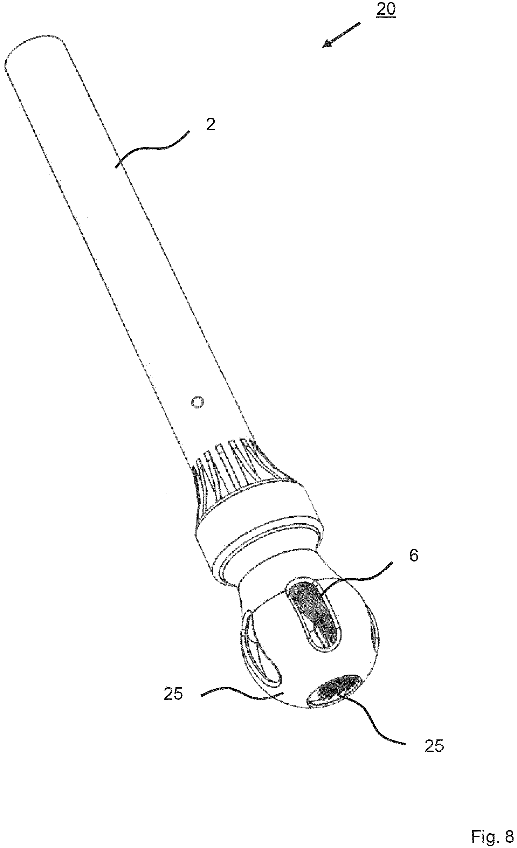

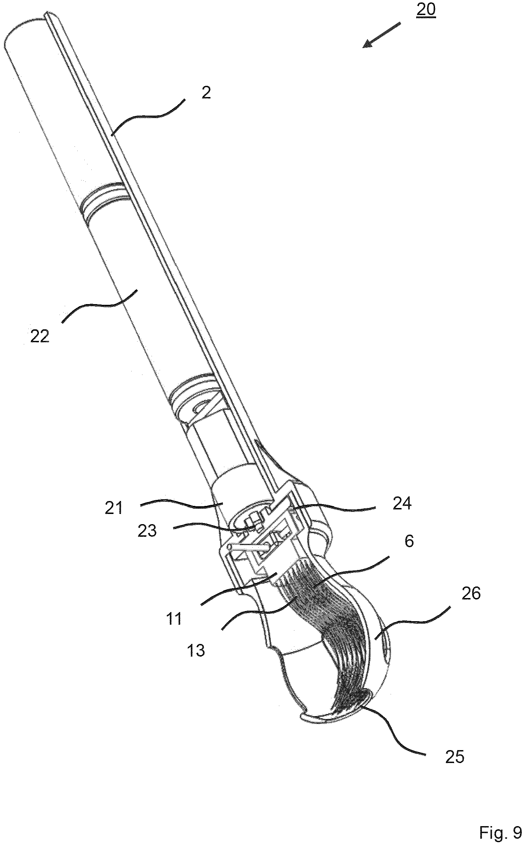

An dem Basisbauteil 4 ist ein Handstück 7 gebildet, welches vom Nutzer mit den Fingern gegriffen werden kann, um Stechnadelspitzen 8 der Stechnadeln 6 zur Microneedling-Behandlung auf eine zu behandelnde Hautoberfläche (nicht dargestellt) aufzusetzen. Hierbei biegen sich die Stechnadeln 6 (vgl. Fig. 8 und 9 unten). Mit den Fingern und der Hand kann der Nutzer dann das Handstück 7 bewegen, beispielsweise derart, dass eine Kreiselbewegung um den Aufsetzpunkt auf der zu behandelnden Haut ausgeführt wird, wobei die Stechnadeln 6 hierbei in der jeweils gekrümmten oder gebogenen Stellung verbleiben.

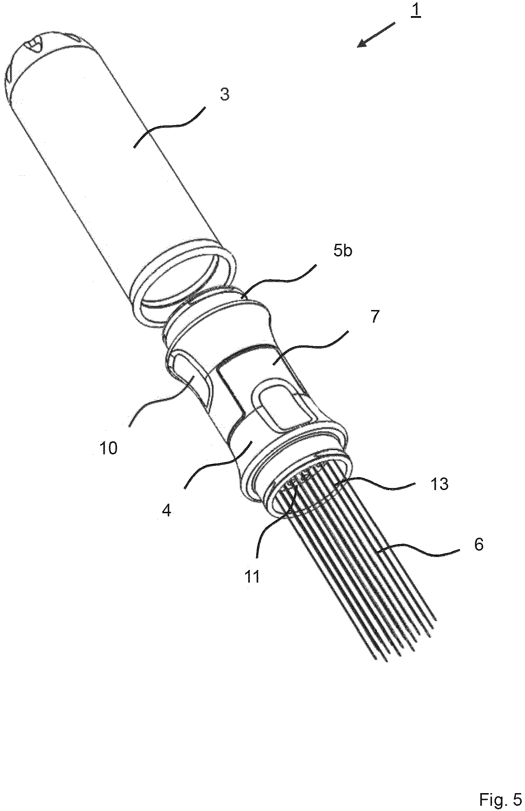

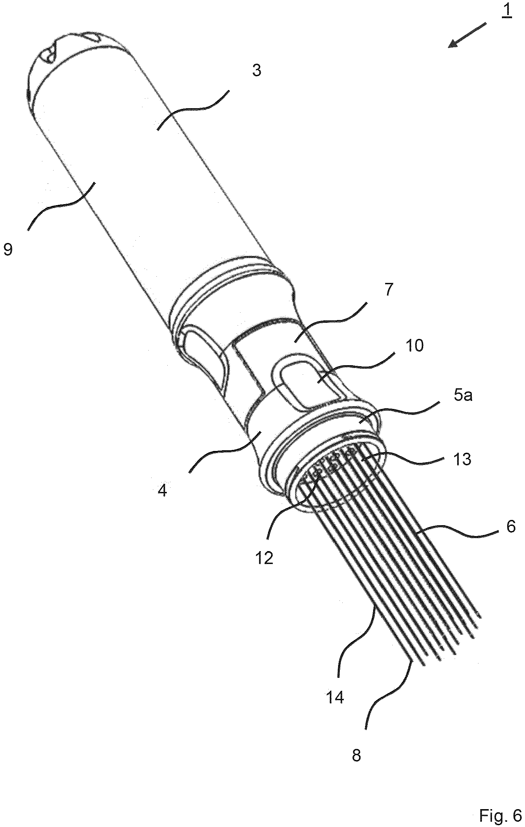

Gemäß einer alternativen Ausgestaltung in den Fig. 5 und 6, kann die Schutzkappe 3 rückseitig an einem Montageabschnitt 5b auf das Basisbauteil 4 aufgesetzt werden, beispielsweise mittels Aufschraubens oder Aufsteckens, so dass eine (zum Beispiel rückwärtige) Verlängerung 9 für das Handstück 7 ausgebildet ist, was eine sichere Haltung des Microneedling-Handgeräts 1 in der Hand des Nutzers unterstützt. Die Schutzkappe 3 ist bei der gezeigten Ausführungsform also insbesondere dahingehend multifunktionell ausgebildet, dass sie einerseits im Nicht-Betriebszustand die Anordnung von Stechnadeln 6 abdeckt und andererseits im Betriebszustand als ein Handstück vergrößernd oder allein ausbildend nutzbar ist.

An dem Handstück 7 sind Fingergriffflächen 10 vorgesehen.

Gemäß den Fig. 2 bis 4 sind die Stechnadeln an einem Nadelträger 11 im Bereich eines Flächenabschnitts 12 des Nadelträgers 11 beabstandet voneinander angeordnet. Hierbei ist ein in Bezug auf den Nadelträger 11 proximales Nadelende 13 im Bereich des Flächenabschnitts 12 fixiert, wohingegen distale Nadelenden 14 mit der zugeordneten Stechnadelspitze 8 freiliegend (nicht fixiert) ausgebildet sind.

Die Stechnadeln 6 sind aus einem Vollmaterial gebildet, wobei als Werkstoff beispielsweise Metall oder Kunststoff zum Einsatz kommen kann.

Die fixierte Lagerung der proximalen Nadelenden 13 und die Dreh- oder Schwenkbarkeit der distalen Nadelenden 14 nach dem Aufsetzen der Stechnadelspitze 8 auf die zu behandelnde Haut bedingt ein Knick- oder Beugungsverhalten der Stechnadeln 6, welches dem sogenannten 3. EULERschen Knickfall entspricht. Das proximale Nadelende 13 ist quasi eingespannt oder fixiert, wohingegen das distale Nadelende 14 der Stechnadel mit der Stechnadelspitze 8 auf einer Hautoberfläche (nicht dargestellt) gelenkig gelagert ist. Für diese Art des Knickverhaltens gilt Folgendes:

i) mit einer kritischen Knicklast $F_{kritisch}$

$$F_{kritisch} = \frac{2\pi^2}{L^2} \cdot I \cdot E$$

und

ii) mit einer Knicklänge s

$$s = \frac{1}{2}\sqrt{2} \cdot L \,,$$

wobei L die Nadellänge der Stechnadel, E das Elastizitätsmodul für ein Material der Stechnadel und *I* das axiale Flächenträgheitsmoment des Querschnitts der Stechnadel angeben.

Hierbei kann ein Verhältnis aus im Betrieb tatsächlich auftretender (Knick-)Last $F_{real}$ und kritischer Knicklast von ≤ 1 vorgesehen sein (($F_{real}$ / $F_{kritisch}$) ≤ 1), vorzugsweise deutlich darunter.

Die Schutzkappe 3 weist im Bereich eines vorderen Endes 15 Durchbrüche 16 auf, die nutzbar sind, um die Stechnadeln 6 wahlweise bei aufgesetzter Schutzkappe 3 vor und / oder nach einer Nutzung für die Hautbehandlung zu sterilisieren.

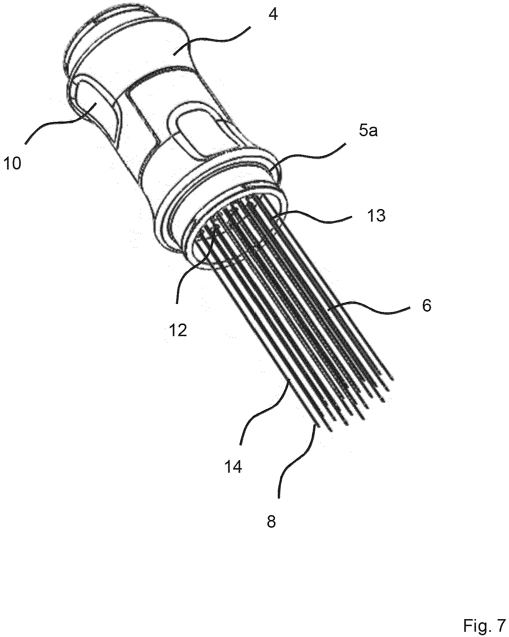

Schließlich zeigt Fig. 7 eine alternative Ausgestaltung eines Microneedling-Handgeräts ohne Schutzkappe 3.

In den Fig. 8 bis 10 ist ein anderes Microneedling-Handgerät 20 in maschinell angetriebener Ausführung gezeigt. Für die gleichen Merkmale werden in den Fig. 8 bis 10 dieselben Bezugszeichen wie in den Fig. 1 bis 7 verwendet.

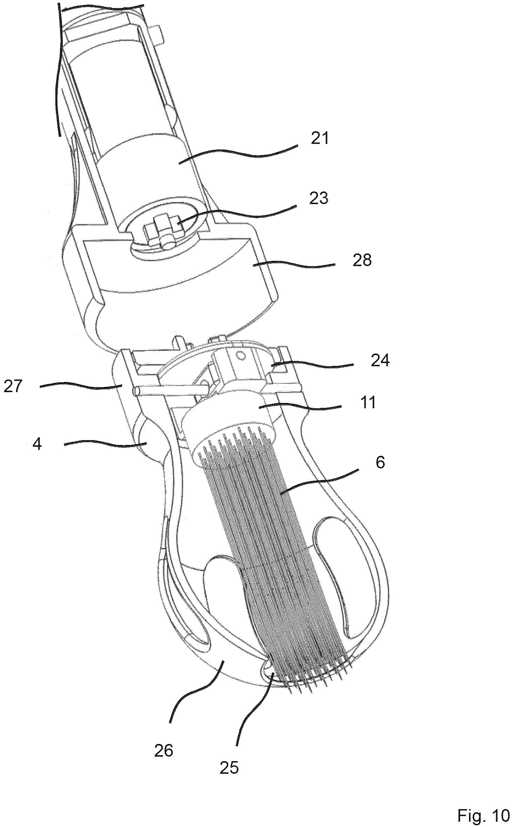

Gemäß Fig. 8 ist in dem das Handstück bereitstellenden Gehäuse 2 eine Antriebseinrichtung 21 in Form eines elektrischen Motors angeordnet, welcher bei der gezeigten Ausführungsform Energie von Batterien 22 empfängt. Die Antriebseinrichtung 21 weist vorderseitig ein Exzenterbauteil 23 auf, welches an den Nadelträger 11 mit der Anordnung von Stechnadeln 6 koppelt, derart, dass der Nadelträger 11 aufgrund der eingekoppelten Antriebskraft und einer Zwangs-Gleitführung 24 in xy-Richtung eine ellipsen- oder kreisförmige Bewegung frei von einer Rotation in einer Bewegungsebene quer zur Längsrichtung des Gehäuses 2 ausführt. Die Fig. 8 und 9 zeigen, teilweise in Schnittdarstellung, für die Stechnadeln 6 eine Situation, wenn das andere Microneedling-Handgerät 20 im Bereich einer vorderen Öffnung 25 einer Nadelführungskappe 26 auf eine zu behandelnde Hautfläche (nicht dargestellt) aufgesetzt ist. Hierbei biegen sich dann die Stechnadeln 6 elastisch und flexibel innerhalb der Nadelführungskappe 26, wie dies in den Fig. 9 und 10 gezeigt ist. Wird sodann der Nadelträger 11 mit den hieran fixierten proximalen Nadelenden 13 aufgrund der eingeleiteten Antriebskraft in der Bewegungsebene quer zur Längsrichtung bewegt, laufen die proximalen Nadelenden 13 der Stechnadeln 6 in Nadellängsrichtung quasi auf einer Kreisbahn um den jeweiligen Einstechpunkt um.

Im nicht auf die Hautoberfläche aufgesetzten Zustand sind die Stechnadeln 6 gemäß dem gezeigten Ausführungsbeispiel in Fig. 10 nicht gebogen (gerade). Demgegenüber zeigen die Fig. 8 und 9 die Anordnung der dann gebogenen Stechnadeln 6 im aufgesetzten Zustand.

Mittels einer wahlweise vorgesehen Einrichtung (nicht dargestellt), die innerhalb der Öffnung 25 angeordnet sein kann, können die Stechnadeln 6 einem Anordnungsmuster entsprechend geordnet werden, dass die Stechnadelspitzen 8 beim Aufsetzen auf die zu behandelnde Hautfläche einerseits das (aufgeprägte) Anordnungsmuster der Stechnadeln 6 wiederholen und andererseits zum Ende des Aufsetzvorgangs annähernd in einer gedachten Mitte der umlaufenden Kreisbewegung zum Liegen kommen. Bei dieser Ausführungsform können die Stechnadeln 6 auch in der nicht auf die behandelnde Hautfläche aufgesetzten Stellung leicht gebogen sein.

Stechnadelspitzen 8 sind auch in der (nicht auf die zu behandelnde Hautfläche aufgesetzten) Stellung der Stechnadeln 6 gemäß Fig. 10 außerhalb vor der Öffnung 25 angeordnet. Fig. 10 zeigt weiterhin eine wahlweise vorgesehene Lösbarkeit des Basisbauteils 4 (Wechselbarkeit). Hierzu ist ein rückseitiger Abschnitt 27 des Basisbauteils 4 in einer zugeordneten Aufnahme 28 lösbar montierbar, zum Beispiel mittels einer Steck- und / oder einer Schraubverbindung.

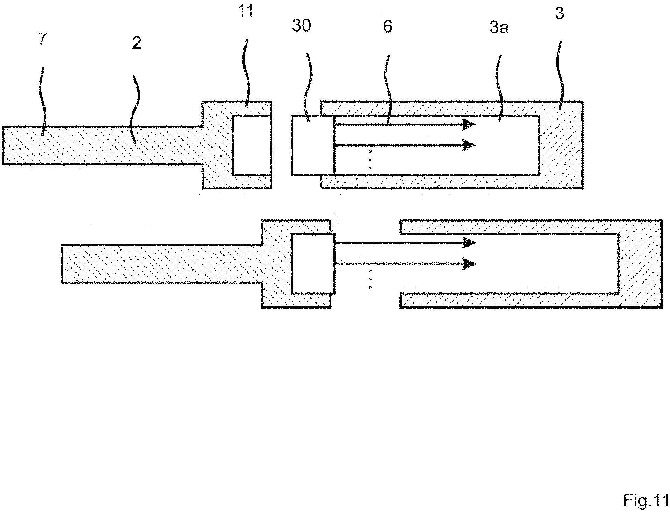

Die Fig. 11 bis 13 zeigen schematisch weitere Ausführungsformen für ein manuelles Microneedling-Handgerät zum lokalen Aufstechen einer Haut. Gezeigt sind jeweils Schnittdarstellungen. Für gleiche Merkmale werden dieselben Bezugszeichen wie in den vorangehend erläuterten Figuren verwendet.

Bei den alternativen Ausführungsformen ist jeweils die Schutzkappe 3 vorgesehen, die lösbar an dem Basisbauteil 4 angebracht ist und im aufgesetzten Zustand im Innenraum 3a die Stechnadeln 6 aufnimmt.

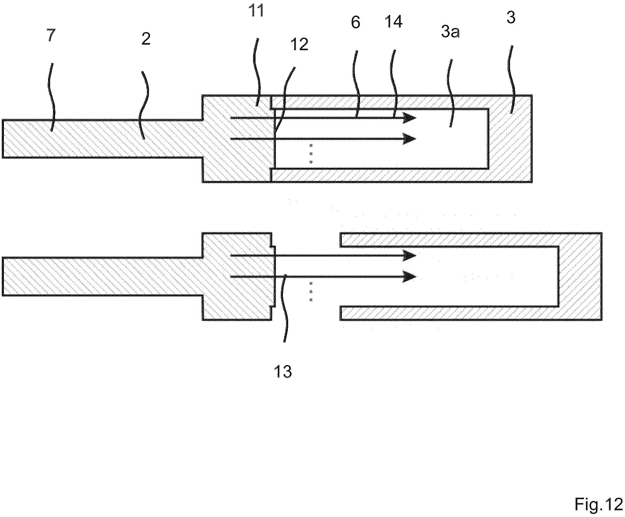

Die Stechnadeln 6 sind bei den Ausführungsformen in den Fig. 11 und 12 jeweils an einem Nadeltragelement 30 fixiert, welches seinerseits bei der Ausführungsform in Fig. 11 lösbar an dem Nadelträger 11 angeordnet ist. Demgegenüber sind die Stechnadeln 6 bei der Ausführungsform in Fig. 12 am Nadelträger selbst angeordnet, also frei von dem Nadeltragelement 30.

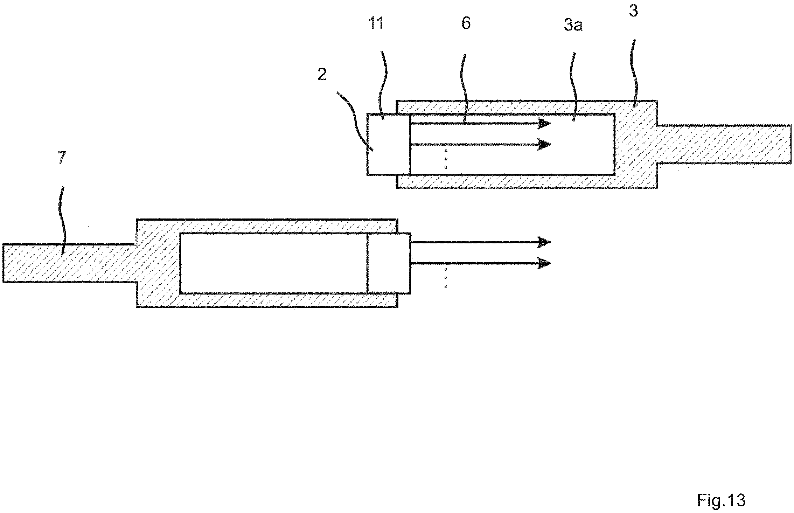

Bei der Ausführungsform in Fig. 13 ist die Schutzkappe 3 multifunktionell ausgebildet und bildet zunächst am Basisbauteil 2 mit Nadelträger 11 die Schutzkappe und nach dem Umstecken rückseitig das Handstück 7.

An der Schutzkappe 3 und / oder dem Handstück 7 können funktionelle Elemente vorgesehen sein. Beispielsweise kann eine optische Lupe vorgesehen sein. Alternativ oder ergänzend kann an der Schutzkappe 3 und / oder dem Handstück 7 ein Spatel, wenigstens mindestens eine Spitze, ein Hautstimulator und / oder ein anderes Werkzeug vorgesehen sein, insbesondere zur Hautbehandlung und / oder -manipulation. Auch eine oder mehrere Aufnahmen für eine Flüssigkeit, die vor, während und / oder nach einer Microneedling-Behandlung zu applizieren ist, sind denkbar, ebenso alternativ oder ergänzend ein Werkzeug oder steriles Zubehör, beispielsweise Zahnstocher, feines Garn oder eine Schlaufe für Haarentfernung oder Hautpflege oder dergleichen.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

**Patentansprüche**

1. Microneedling-Handgerät (1; 20) zum lokalen Aufstechen einer Haut, mit

   - einem von einem Nutzer greifbaren Handstück (7) und
   - einer Microneedling-Hautstecheinrichtung, die einen Nadelträger (11) mit einer Anordnung von Stechnadeln (6) aufweist, bei der über einen Flächenabschnitt (12) des Nadelträgers (11) verteilt mit Abstand voneinander mehrere flexibel biegsame Stechnadeln (6) angeordnet sind, wobei ein in Bezug auf den Flächenabschnitt (12) proximales Nadelende (13) der Stechnadeln (6) an dem Nadelträger (11) fixiert ist und die Stechnadeln (6) jeweils aus einem Vollmaterial bestehen.

2. Microneedling-Handgerät (1; 20) nach Anspruch 1, **dadurch gekennzeichnet, dass** die Stechnadeln (6) einen Durchmesser von höchstens etwa 0,20 mm aufweisen.

3. Microneedling-Handgerät (1; 20) nach Anspruch 1 oder 2, **dadurch gekennzeichnet, dass** die Stechnadeln (6) einen im Wesentlichen kreisrunden Querschnitt aufweisen.

4. Microneedling-Handgerät (1; 20) nach mindestens einem der vorangehenden Ansprüche, **dadurch gekennzeichnet, dass** die Stechnadeln (6) voneinander einen Abstand von wenigstens etwa 1 mm aufweisen.

5. Microneedling-Handgerät (1; 20) nach mindestens einem der vorangehenden Ansprüche, **dadurch gekennzeichnet, dass** die Stechnadeln (6) eine Nadellänge zwischen etwa 1 mm und etwa 100 mm aufweisen.

6. Microneedling-Handgerät (1; 20) nach mindestens einem der vorangehenden Ansprüche, **dadurch gekennzeichnet, dass** die Anordnung von Stechnadeln (6) wenigstens fünf Stechnadeln (6) im Bereich des Flächenabschnitts (12) aufweist.

7. Microneedling-Handgerät (1; 20) nach mindestens einem der vorangehenden Ansprüche, **dadurch gekennzeichnet, dass** jeweilige Längsachsen der Stechnadeln (6) zumindest in einer nichtgebogenen Stellung der Stechnadeln (6) im Wesentlich parallel zueinander verlaufen.

8. Microneedling-Handgerät (1; 20) nach mindestens einem der vorangehenden Ansprüche, **dadurch gekennzeichnet, dass** die Stechnadeln (6) in der nichtgebogenen Stellung einer Bauform aus der folgenden Gruppe entsprechend gebildet sind: gerade Nadel, schlangenlinienförmige Nadel und gekrümmte Nadel.

9. Microneedling-Handgerät (1; 20) nach mindestens einem der vorangehenden Ansprüche, **dadurch gekennzeichnet, dass** die Microneedling-Hautstecheinrichtung mit dem greifbaren Handstück (7) lösbar verbunden ist.

10. Microneedling-Handgerät (1; 20) nach mindestens einem der vorangehenden Ansprüche, **dadurch gekennzeichnet, dass** der Nadelträger (11) lösbar an der Microneedling-Hautstecheinrichtung angeordnet ist.

11. Microneedling-Handgerät (1; 20) nach mindestens einem der vorangehenden Ansprüche, **dadurch gekennzeichnet, dass** zumindest distale Stechenden (14) der Stechnadeln (6) in einer Schutzkappe (3) angeordnet sind, welche lösbar ist.

12. Microneedling-Handgerät (1; 20) nach Anspruch 11, **dadurch gekennzeichnet, dass** die Schutzkappe (3) nach dem Lösen und dem Freigeben der distalen Stechenden (14) rückseitig an der Microneedling-Hautstecheinrichtung anordenbar und so das greifbare Handstück (7) zumindest teilweise ausbildend ausgeführt ist.

13. Microneedling-Handgerät (1; 20) nach Anspruch 11 oder 12, **dadurch gekennzeichnet, dass** die Schutzkappe (3) ein oder mehrere Durchbrüche (16) aufweist, welche für eine Sterilisation der Stechnadeln (6) durch den einen oder die mehreren Durchbrüche (16) hindurch nutzbar sind.

14. Microneedling-Handgerät (1; 20) nach mindestens einem der vorangehenden Ansprüche, **gekennzeichnet durch** eine Antriebseinrichtung (21), die

   - in einem Gehäuse (2) angeordnet und eingerichtet ist, eine Antriebskraft bereitzustellen, und
   - mit der Microneedling-Hautstecheinrichtung verbunden ist, derart, dass der Nadelträger (11) mit der Anordnung von Stechnadeln (6) mittels der auf den Nadelträger (11) eingeleiteten Antriebskraft eine ellipsen- oder kreisförmige Bewegung in einer Bewegungsebene quer zur Längsachse der Stechnadeln (6) ausführt.

15. Microneedling-Hautstecheinrichtung für ein Microneedling-Handgerät (1; 20) nach mindestens einem der vorangehenden Ansprüche, welche als Wechselmodul ausgeführt ist und die mit einem Nadelträger (11) mit einer Anordnung von Stechnadeln (6) gebildet ist, bei der über einen Flächenabschnitt (12) des Nadelträgers (11) verteilt mit Abstand voneinander mehrere flexibel biegsame Stechnadeln (6) angeordnet sind, wobei ein in Bezug auf den Flächenabschnitt (12) proximales Nadelende (13) der Stechnadeln (6) an dem Nadelträger (11) fixiert ist und die Stechnadeln (6) jeweils aus einem Vollmaterial bestehen.

Fig. 1

Fig. 2

1

4

5a

13

10

12

11

6

14

8

3

Fig. 3

Fig. 4

Fig. 5

Fig. 6

Fig. 7

Fig. 8

20

2

22

21

23

24

6

11

13

26

25

Fig. 9

21

23

28

24

27

11

4

6

26

25

Fig. 10

Fig.11

Fig.12

Fig.13

Europäisches
Patentamt

European
Patent Office

Office européen
des brevets

**EUROPÄISCHER RECHERCHENBERICHT**

**Nummer der Anmeldung**

**EP 22 16 0536**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2016/256674 A1 (SCHERKOWSKI DIRK [DE]) 8. September 2016 (2016-09-08) * Absätze [0007] – [0010], [0044] – [0047]; Abbildungen 8-10 * ----- | 1,3-15 | INV. A61M37/00 |
| X | US 6 030 404 A (LAWSON ALEXIS A [US] ET AL) 29. Februar 2000 (2000-02-29) * das ganze Dokument * ----- | 1,3,5-8 | |
| X | KR 101 236 525 B1 (SHIN YOUNG KYUN [KR]) 22. Februar 2013 (2013-02-22) * Siehe Absätze [0001], [0028] – [0031], [0036], [0046], [0056], [0057] der Übersetzung; Abbildungen 1-7 * ----- | 1,2,5-8 | |
| X | KR 100 978 561 B1 (SHIN YOUNG KYUN [KR]) 27. August 2010 (2010-08-27) * Siehe die Zusammenfassung sowie die Absätze [0030] – [0032], [0056] der Übersetzung; Abbildungen 1-6 * ----- | 1,5-8 | RECHERCHIERTE SACHGEBIETE (IPC) A61M |

Der vorliegende Recherchenbericht wurde für alle Patentansprüche erstellt

| Recherchenort | Abschlußdatum der Recherche | Prüfer |
|---|---|---|
| Berlin | 6. September 2022 | Berndorfer, Urs |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 22 16 0536

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-09-2022

| Im Recherchenbericht angeführtes Patentdokument | Datum der Veröffentlichung | Mitglied(er) der Patentfamilie | Datum der Veröffentlichung |
|---|---|---|---|
| US 2016256674 A1 | 08-09-2016 | BR 102016004656 A2<br>EP 3064251 A1<br>ES 2780876 T3<br>PL 3064251 T3<br>US 2016256674 A1 | 06-09-2016<br>07-09-2016<br>27-08-2020<br>29-06-2020<br>08-09-2016 |
| US 6030404 A | 29-02-2000 | KEINE | |
| KR 101236525 B1 | 22-02-2013 | KEINE | |
| KR 100978561 B1 | 27-08-2010 | KEINE | |

Für nähere Einzelheiten zu diesem Anhang : siehe Amtsblatt des Europäischen Patentamts, Nr.12/82

EPO FORM P0461